Europäisches Patentamt

(19) European Patent Office

Office européen des brevets

(11) Publication number: **0 014 802**
**B1**

# EUROPEAN PATENT SPECIFICATION

(45) Date of publication of patent specification: **21.03.84**

(21) Application number: **79302840.8**

(22) Date of filing: **10.12.79**

(51) Int. Cl.³: **B 01 J 25/04,**
**B 01 J 23/94,**
**C 07 C 85/08, B 03 C 1/00**

(54) Liquid phase chemical process with separation of catalyst particles by magnetic flocculation.

(30) Priority: **21.12.78 GB 4961578**

(43) Date of publication of application:
**03.09.80 Bulletin 80/18**

(45) Publication of the grant of the patent:
**21.03.84 Bulletin 84/12**

(84) Designated Contracting States:
**BE CH DE FR GB IT NL**

(56) References cited:
**GB - A - 1 107 627**
**US - A - 1 390 688**
**US - A - 4 021 367**

(73) Proprietor: IMPERIAL CHEMICAL INDUSTRIES
PLC
Imperial Chemical House Millbank
London SW1P 3JF (GB)

(72) Inventor: **Wood, Laurie**
**5, Lowcross Drive**
**Great Broughton Middlesbrough, Cleveland (GB)**
Inventor: **Lindley, John**
**31, Mackie Drive**
**Guisborough, Cleveland (GB)**

(74) Representative: **Martin, David Lincoln et al,**
**Imperial Chemical Industries PLC Legal**
**Department: Patents Po Box 6**
**Welwyn Garden City Herts, AL7 1HD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Liquid phase chemical process with separation of catalyst particles by magnetic flocculation

The present invention relates to catalytic processes in particular to catalytic processes using supported or unsupported metallic catalysts.

The use of metal catalysts extends throughout all branches of the chemical industry. Processes in which they are used may be homogeneous or heterogeneous with respect to the catalyst and may take place in the vapour phase or liquid phase. It is with this latter type of heterogeneous process that the present invention is concerned.

At the end of a liquid phase process using a heterogeneous catalyst the solid catalyst must be separated from the liquid phase. The solid-liquid separation usually poses some difficulties, and usually involves some form of filtration, with or without prior decantation. For example, the catalyst may be allowed to settle under gravity and the liquid reaction products then decanted or filtered from the solid. In some cases, finely divided solid catalysts or catalysts suspended in a reaction medium may settle rather slowly and this can involve a waste of time while settling takes place. In many processes, for example catalytic hydrogenations, dehydrogenations, desulphurisations, the catalyst used is a ferromagnetic material, for example Raney nickel, in the form of small particles. Where separation of such ferromagnetic catalyst particles from the reaction medium has proved difficult, it has been proposed to use a magnetic filter in the form of a grid or screen, a magnetic field being induced in the grids by an adjacent field coil. The ferromagnetic particles are removed from the liquid reaction medium by causing it to pass through the grid or screen, the particles being retained on the grid or screen. Another proposal for separating difficultly-separable non-magnetic particles from a liquid medium involves the absorption or collection of the non-magnetic particles into or on a suitable added ferromagnetic material and then separating the ferromagnetic material, together with the non-magnetic particles which it has collected, by magnetic filtration. Both these proposals have some disadvantages, for example blocking of the grids or screens by collected material in the one case and the necessity to introduce an extra step involving addition of another material which is then immediately removed in the other case.

In U.S. Patent No. 1,390,688 a method is described for removing catalytic material from fatty oils. In particular the patent describes the removal of finely divided nickel or magnetizable nickel oxide from fatty oils by means of a suitable magnet. In the method described, a mixture of hydrogenated oil and catalyst is removed from a hydrogenation reactor to another vessel or tank which has a number of magnets fitted below it. The tank may be made of aluminium and the magnets may be in actual contact with the bottom of the tank. Under the influence of the magnetic field, the catalyst particles are attracted to and collect on the bottom of the tank adjacent to the magnets. After all the oil has passed through the magnetic zone the magnets are switched off, the catalyst is removed from the tank and recycled to the reactor for further use.

We have now devised a process whereby certain supported or unsupported magnetic or magnetisable metal catalysts may be readily removed from a liquid reaction medium.

Accordingly the present invention comprises a process of conducting a liquid phase chemical reaction carried out in a reactor in the presence of solid particles of a supported or unsupported catalyst comprising a magnetic or magnetisable metal and in which, at the end of said reaction, said catalyst particles are separated from said liquid phase using a magnetic field, characterised in that at the end of the reaction

(1) the catalyst particles in the liquid reaction mixture are magnetically flocculated,
(2) the thus flocculated particles are allowed to settle in the reactor whereby to produce a supernatant liquid phase, and
(3) the supernatant liquid phase is withdrawn from the reactor leaving flocculated and settled catalyst particles in situ in the reactor.

The catalyst may comprise either a magnetic or magnetisable metal and the term "magnetic" is to be construed as including both classes. Thus, the expression comprises both catalysts which are inherently magnetic and catalysts which are capable of being magnetised, at least momentarily, during passage through a magnetic field.

The invention is particularly applicable to processes in which the catalyst comprises one of the ferromagnetic metals, i.e. iron, cobalt, or nickel. The catalyst is used in particulate form and although it may be added as a compound of one of the metals e.g. as the carbonate, hydroxide, oxide or alkanoate, e.g. oxalate or acetate it is customary to convert these compounds to the oxide which may then be reduced in situ to the metal. In another embodiment, the metal may be added as such, for example as a ferromagnetic powder, a particular preferred form of the catalyst being Raney cobalt, Raney iron or Raney nickel, in which the metal is present in finely particulate form presenting a large catalytic surface area. In a further embodiment of the invention, the catalyst may be a magnetic metal on a support, for example nickel on a kieselguhr support.

The three metals mentioned above are extensively used as catalysts in reactions in which an organic compound is reacted in the liquid phase, for example hydrogenation and dehydrogenation reactions, isomerisation, disproportionation, reductive amination, halogenolytic dissociations, for example dehalogenation, debenzylation. Typical hydrogenation reactions include the hydrogenation of olefines, acetylenes and aromatic compounds e.g. benzene to cyclohexane and phenol to cyclohexanol, the hydrogenation of aldehydes and acids to alcohols, and oximes, nitriles, imines and nitro- compounds to amines, complete hydrogenation of the double bonds of unsaturated fats and fatty acids, partial hydrogenation of fats and oils with multiple unsaturation ("hardening" of fats), hydrogenation of glucose to sorbitol and mannitol. Typical dehydrogenation processes include the conversion of alcohols to aldehydes or ketones. Other reactions in which these metals may be used as catalysts include isomerisation, for example of 1-butene to 2-butene, of allyl alcohol to propionaldehyde, and the hydrogenation of glyceride oils to unsaturated fatty acid ester isomers; disproportionation, for example the conversion of primary amine to secondary amine with release of ammonia; reductive amination, for example of alcohols, aldehydes, ketones or nitro- compounds to form amines; dehalogenation; debenzylation (dearomatisation); hydrogenolysis; desulphurisation; homologation, by increasing the chain length of, for example methanol to form ethanol. A typical reaction in which a metal catalyst such as Raney nickel may be used is the reductive amination of an aldehyde or alcohol to an amine. The aldehyde or alcohol e.g. a $C_2$ to $C_{20}$ aliphatic aldehyde or alcohol may be reacted with ammonia or with a primary or secondary amine e.g. dimethylamine to give a primary, secondary or tertiary amine respectively. These reductive amination processes may be carried out at pressures up to 200 atmospheres of hydrogen, at temperatures of 20° to 200°C and at catalyst concentrations of up to 10% w/w preferably up to 5% w/w of the material to be hydrogenated.

Although in many embodiments of the process according to the invention the material to be reacted provides the reaction medium, in some instances it is preferable to have a solvent for example an alcohol, or diluent present to maintain the liquid phase and/or to decrease the viscosity of the liquid medium. An appropriate solvent or diluent may also serve to absorb water of reaction when that is formed. For example, in the hydrogenation processes referred to above the reaction medium may comprise a saturated hydrocarbon e.g. a $C_6$ to $C_{16}$ alkane or cycloalkane, or an alcohol.

The magnet used to effect the magnetic flocculation may be a permanent magnet (which can be moved inwards physically toward the reaction vessel when magnetic flocculation is required) but is preferably an electromagnet. The electromagnet should be located so that the catalyst particles are readily flocculated to form agglomerates which can settle or be directed towards a convenient removal point in the reactor in which the catalytic reaction is taking place, for example at the base of the reactor. The electromagnet is conveniently of toroidal shape and is preferably powered by D.C. current. The electromagnet may be positioned externally of the reactor or within the reactor. If the electromagnet is fitted externally, the reactor must be constructed from a non-magnetic material, for example stainless steel. If fitted internally, it is desirable to protect the electromagnet from the process fluid with a suitable protective cover but considerable capital savings can be made compared with external fitting of the electromagnet by the use of carbon steel for the reactor pressure vessel (provided carbon steel is inert to the process liquors).

If desired, the reactor may be fitted with a stirrer and/or other agitators. It is particularly desirable to use agitators specifically designed for efficient gas/liquid contacting when a gas is used in the process of the invention.

The liquid phase process of this invention may be carried out either continuously or on a batch basis. If desired, the batch process may be operated on a semicontinuous basis using a pumped feed so that the process liquor volume in the reactor increases as reaction progresses until a predetermined maximum liquor volume is reched at the end of the batch. At the end of the process, the magnetic field is applied to the reaction mixture to cause magnetic flocculation of the catalyst particles. The magnetic flocculation is effected without the need to add supplementary magnetic or non-magnetic material. Application of the magnetic field causes flocculation of the catalyst particles into clumps consisting of large numbers of individual particles with the result that the clumps settle to the bottom of the reactor vessel under the influence of gravity. Using the preferred toroidal magnet, the catalyst particles are attracted towards the centre of the coil, that is to the middle of the reactor vessel where they flocculate to form agglomerates although there may be some attraction also to the magnetic coil itself.

The magnetic field may be applied continuously to the reaction mixture at the end of the process but we have found the continuous application is not essential in all cases. The application may be intermittent and so timed as to cause periodic acceleration in the downward movement of the catalyst particles. Suitably the magnetic fluid applied is at least 800 Amperes/metre (10 oersted) preferably at least 4000 Amperes/metre (50 oersted), particularly in the range 4000 to 400,000 Amperes/metre (50 to 5000 oersted).

When magnetic flocculation and settling of the catalyst is complete, the supernatant liquid product can be removed from the reactor, for example, by being blown out along a product conduit by residual reactor pressure, leaving the catalyst behind. The catalyst can be either used again or separately removed from the reactor, for example by reslurrying it in water or a solvent and then pumping or draining out of the reactor.

As a precaution against any small amounts of catalyst which may be removed from the reactor with the liquid phase, it is preferred to pass the liquid phase through a "back-up" or "guard" filter which can conveniently be located in the liquid product exit conduit from the reactor. The "guard" filter is preferably a high gradient magnetic separator (HGMS) but other filters, for example a back-flushable stainless steel radial fin filter element, a cartridge filter and plate and frame filters are also suitable. If an HGMS separator is used, it is preferred to back-flush it with at least a portion of the new reaction feedstock when it is fed to the reactor for resumption of the process.

The following Examples further illustrate the process of the invention and apparatus for carrying it out. The reaction specifically illustrated with reference to the drawings is the amination of aldehydes using a Raney nickel catalyst but it is emphasised that this is merely one illustration of the many reactions (as hereinbefore mentioned), which employ magnetic catalysts and to which the process of this invention is generally applicable, for example hydrogenations, dehydrogenations, isomerisations, disproportionations etc.

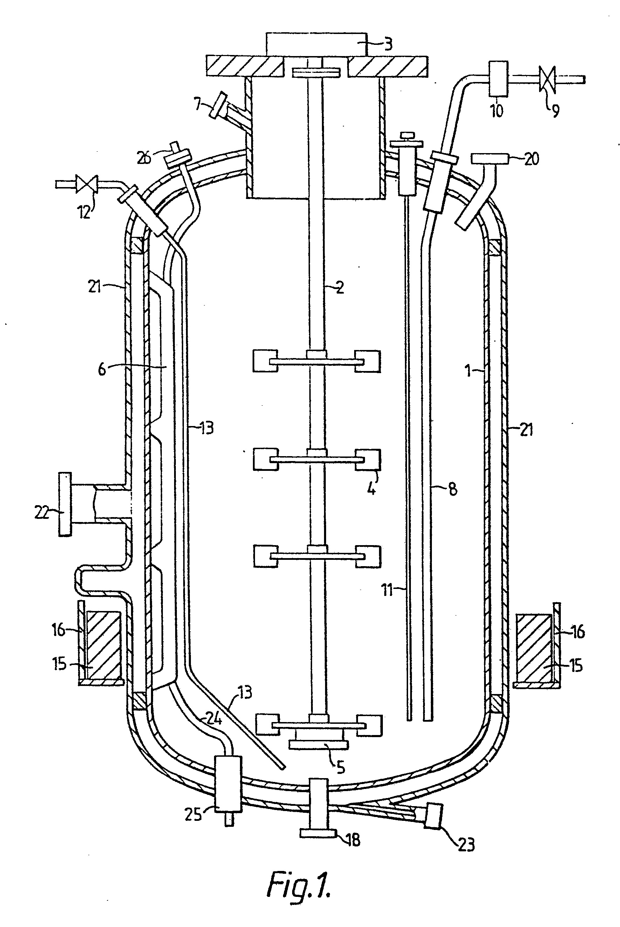

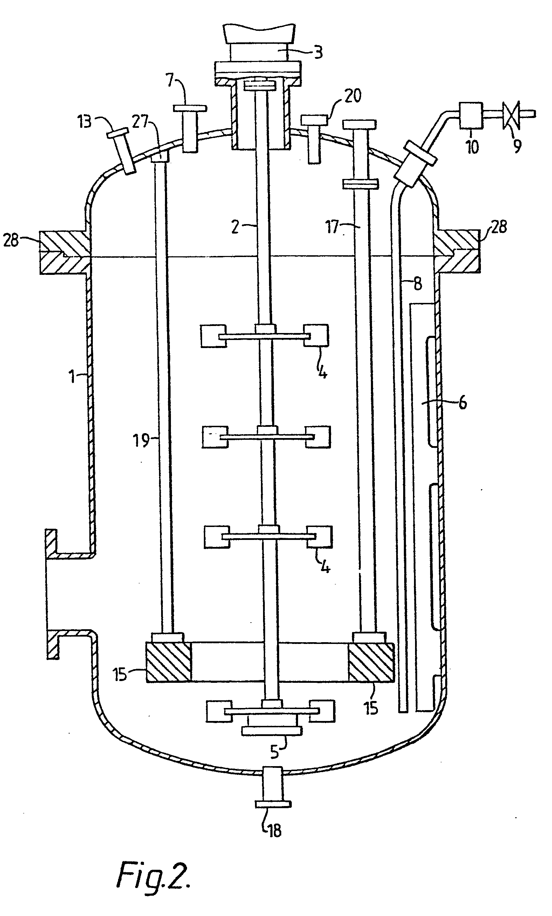

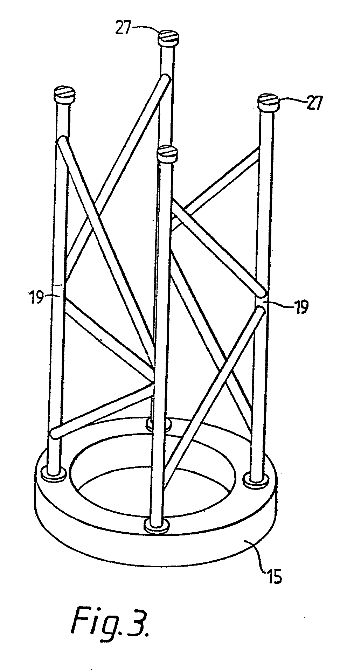

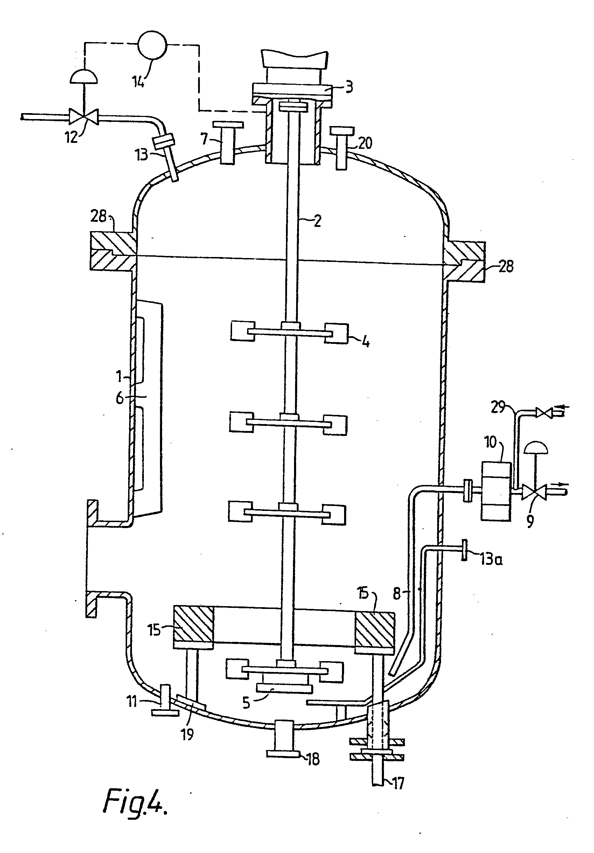

Figure 1 is a diagrammatic sketch, partly in section, of a reactor for the process of the invention fitted with an external electromagnet. Figures 2 and 4 are similar views of reactors fitted with internal electromagnets. Figure 3 illustrates the support unit for the electromagnet coil used in the reactor of Figure 2 and is on a somewhat smaller scale than Figure 2. In the drawings, similar items of equipment have been allotted similar identifying reference numbers in all three drawings.

Referring to Figure 1, a cylindrical reactor 1 in the form of a pressure vessel and made of stainless steel has a central rotatable agitator shaft 2 passing into the reactor through a gland and seal assembly 3 and fitted with a series of turbine impellers 4 along its length, and with an agitator stabilising ring 5 at the bottom of the shaft. For vertical baffles 6 are fitted at intervals around the walls of the reactor. Liquid reactants enter the reactor 1 through line 7. Line 8, which extends to near the base of the reactor, is the outlet for the liquid products of the process. Flow along line 8 is controlled by a valve 9 and a high gradient magnetic separator 10 is positioned on the reactor side of this valve. The level of liquid in the reactor is monitored by a suitable level detector 11, for example an ultrasonic detector. Reactant gas can be fed to the reactor 1 through valve 12 and line 13 and a pressure controller 14 (not shown in Figure 1) is connected to this valve.

A D.C. powered electromagnet 15 is fitted around reactor 1 at a position close to the base of the reactor and is protected by a carbon steel magnetic shield 16. The electromagnet is provided with services, for example water through services conduit 17 (not shown in Figure 1). In operation, it is essential to guard against power being supplied to electromagnet 15 and magnetic separator 10 without their associated cooling water. To this end, therefore, water from the cooling systems of the electromagnet and separator flows directly to drain with no intervening valve. In addition both a water pressure switch and a water flow switch are provided and are interconnected with the power system supplying the electromagnet and separator in such a way that power will not be supplied unless coolant water is flowing at a minimum pre-set rate. Reactor 1 is fitted with a bottom drain valve 18, and with an inlet 20 for the admission of catalyst. Surrounding reactor 1 is stream jacket 21 with an inlet 22 and condensate outlet branch 23. Reactor 1 is also supplied with an internal cooling pipe 24 supplied with liquid coolant, for example water, through inlet 25. The coolant exits through outlet 26.

Referring to Figures 2 and 3 similar parts are designated with the same numbers as in Figure 1 but for clarity certain items carried by the reactor shown in Figure 2 and already illustrated in Figure 1 have been omitted viz; level detector 11, steam jacket 21, cooling pipe 24. In this embodiment, electromagnet 15 is fitted annularly within reactor 1. The electromagnet is suspended by a framework of support arms 19 (see Figure 3) suspended by pads 27 from the top of reactor 1. (For clarity in Figure 2, only one arm 19 and is associated pad 27 is shown). The electromagnet 15 is supplied with services through conduit 17. Reactor 1 is fitted with flanges 28 thereby allowing the top of the reactor, to which are fitted the various inlet and outlet conduits, agitator etc., to be removed from the reactor as an entity.

Referring to Figure 4, a reactor similar to that in Figure 2 is shown but with a number of differences. Once more, certain items also common to the reactors shown in Figures 1 and 2 have been omitted for clarity. Outlet line 8 is connected, on the upstream side of the separator 10 to an inlet line 29 with associated valve. Inlet line 29 is for incoming liquid reactant or solvent or for water for catalyst washing prior to discharge. The arrangement enables solvent to be back-flushed along line 8 into the reactor once the reactor has been emptied of liquid product along lines 8 and 9 in the usual way.

Reactant gas is fed through valve 12 and line 13 into reactor 1 as described for the reactors shown in Figures 1 and 2 but if desired reactor gas can alternatively or additionally be admitted through line 13a which extends to below the base of agitator 2. The electromagnet 15 has a bottom mounted design support arrangement 19 including a conduit 17 with a packed gland arrangement for the admission of services.

Each of the reactors shown in the accompanying drawings may, if desired, be fitted in the usual way with services such as external steam jacketting for heating purposes and internal cooling coils (as illustrated in Figure 1) but these items, whose form and possible location will be familiar to those skilled in this art have been omitted from the Figures 2 and 4 in the interests of clarity.

One reaction for which the reactors illustrated in Figures 1, 2, 3 and 4 is suitable is the amination of aldehydes using a Raney nickel catalyst. In this process reactor 1 (volume 2.5m³) was purged with nitrogen and then with hydrogen prior to the addition of 25 kg of finely divided Raney nickel catalyst suspended in 25 kg of water through inlet 20. Methanol solvent was added and liquid ammonia charged via the dip pipe 8, passing through the HGMS unit 10 in a reverse direction. The 4-impeller agitator 2 was switched on and adjusted to a speed of 100 rpm. Steam was applied to the heating jacket (see Figure 1) and the reactor contents were raised to a temperature in excess of 100°C and maintained under temperature control. Hydrogen was supplied on pressure control through valve 12 and inlet 13 to the design working pressure. The feedstock injector pump was commissioned and the feedstock was pumped into the reactor through line 7. The heat of reaction was removed using a cooling coil (see Figure 1) as the reaction progressed. At the end of the reaction period the stirrer speed was reduced to minimum (30 rpm) in order to allow the reactor contents to degas and maximum cooling water was applied through the cooling coil 24.

When the reactor had cooled to 60°C, the stirrer was stopped and the magnetic flocculating coil 15 actuated for 2 mins, taking a power of about 45 kw through water cooled conductors supplied through a coil external to the reactor (Figure 1) or through the services lines 17 (Figures 2 and 4). The magnetic field applied was about 47,750 Amperes/metre (600 Oersted). Actuation of the coil 15 may be repeated 2 to 3 times at intervals as necessary, the flocculated catalyst being allowed to settle during each such interval.

After a 20 minute settling time the reactor contents were discharged through the dip pipe 8 over a period of 20 minutes through the HGMS unit 10. During the transfer through the HGMS unit the nickel content of the process liquor was reduced from 20 ppm to less than 1 ppm of suspended nickel particles. The major portion of the catalyst was left in the reactor 1, together with 30—40 grams in the HGMS filter 10. A new batch was then prepared. The incoming methanol and ammonia for the second batch were pumped back through the HGMS unit 10 and dip tube 8 into the reactor containing the 25 kg of catalyst and about 0.12m³ of reactor product remaining from the first batch. The stirrer was recommissioned and the flocculated catalyst which had collected at the base of the reactor was quickly resuspended.

When, eventually, the catalyst is partly or wholly deactivated, it is washed with methanol to remove the amine and ammonia, and these washings are passed to distillation. For each washing, the agitator 2 is used and the electromagnet 15 is actuated to assist the settling of the washed catalyst particles. Finally, the catalyst is washed with water to remove methanol and the catalyst is then discharged into drums containing sodium hypochlorite to finally destroy the pyrophoric nature of the catalyst.

The use of magnetic flocculation substantially reduces the total settling time necessary to achieve a given fractional recovery of settled catalyst (for example 20 minutes as against 2 to 3 hours for conventional gravitational settling). Further evidence of this improvement in settling time is provided by a series of tests on 7 supported catalysts consisting of 6 nickel-on-kieselguhr catalysts in which the amount of nickel ranged from 25% to 68% and one cobalt-on keisulguhr catalyst containing 45% cobalt. Settling tests were carried out in a 250 ml measuring cylinder located between the poles of an electromagnet with a field strength of 47,750 A/m (600 Oersted) centrally and about 23,875 A/m (300 Oersted) at the top and bottom of the cylinder. For comparison, gravity settling tests were also carried out in a zero field.

The concentrations of catalysts used were in the range of 8 to 20 gm/litre. In each case, the field was applied for 3 minutes to effect flocculation and the catalyst particles were then allowed to settle for a further 12 minutes. In the comparative tests with no field, gravity settling was allowed for 15 minutes.

At the end of the respective settling periods, the concentration of catalyst still in suspension was determined and the ratio

concentration of catalyst in suspension without flocculation

―――――――――――――――――――――――――――――――――――

concentration of catalyst in suspension with flocculation

calculated. Depending on the catalyst, this ratio ranged from 2.0 to 6.3 clearly illustrating the effectiveness of magnetic flocculation. In a typical experiment on a 55% nickel-on-kieselguhr commercial catalyst, 98.5% of the catalyst settled out after magnetic flocculation whereas only 94% settled out by gravity in the absence of magnetic flocculation.

Referring to the drawings once more, the effectiveness of the high gradient magnetic separator (HGMS) 10 is illustrated by reference to a typical example in which the reactor 1 contained 1.25 m³ liquid and 25 Kg Raney nickel catalyst. The mixture was stirred for 1 hour and the catalyst was then subjected to magnetic flocculation, as hereinbefore described, for 5 minutes. Thereafter the catalyst particles were allowed to settle for a further 30 minutes. The supernatant liquor was then removed over a period of 18 minutes through line 8 and HGMS unit 10. Samples of the liquor were taken at the inlet and outlet of the HGMS unit. These samples, four of which were taken at each point, showed that the liquor entering the HGMS unit had a nickel concentration in the range of 40 to 50 ppm whereas the liquor leaving the HGMS unit had a much lower nickel content, in the range 0.5 to 3.0 ppm.

The use of the process of this invention enables a compact reactor design to be used which is of some importance when a normally pyrophoric catalyst, for example Raney nickel, is used. In the prior art, similar conventional processes have required several vessels and the transfer from vessel to vessel of the catalyst. The process of this invention allows the use of a compact reactor design (as shown in the accompanying drawings), thereby eliminating transfers from vessel to vessel and resulting in the use of fewer joints and seals which can fail as well as a lower inventory of possibly highly flammable solvent. For the process of this invention, the need for equipment external to the reactor vessel is much reduced compared to the prior art. These factors, therefore, all illustrate the considerable improvement in safety possible by using the process of this invention.

The process of this invention also enables a longer catalyst life to be achieved. The catalyst is not exposed to the atmosphere and there is no opportunity for oxygen to deactivate the catalyst irreversibly. If necessary the catalyst can be maintained continuously under an inert atmosphere, for example hydrogen and this, coupled with the absence of handling and transfer of the catalyst once it has been charged to the reactor, increases catalyst life apprecialby. The absence from the process of the invention of external loops, settling tanks, recycle vessels such as are used in prior art processes has allowed the same catalyst charge to be used for 25 successive batches in the amination of an aldehyde feedstock. A comparative experiment under labotatory conditions using a prior art process without magnetic flocculation but using a separate catalyst recovery vessel was not only much more time-consuming but showed that the catalyst was fit only for 12 successive batches.

Since a relatively simple reactor design is needed for the process of this invention, it follows that less instrumentation is needed and less energy used than in conventional prior art processes. The confining of the catalyst to the reactor during a series of batches also allows catalysts to be used which hitherto have been rejected on grounds of either difficulty of recovery or safety.

**Claims**

1. A process of conducting a liquid phase chemical reaction carried out in a reactor in the presence of solid particles of a supported or unsupported catalyst comprising a magnetic or magnetisable metal and in which, at the end of said reaction, said catalyst particles are separated from said liquid phase using a magnetic field, characterised in that at the end of the reaction

(1) the catalyst particles in the liquid reaction mixture are magnetically flocculated,
(2) the thus flocculated particles are allowed to settle in the reaction whereby to produce a supernatant liquid phase, and
(3) the supernatant liquid phase is withdrawn from the reactor leaving flocculated and settled catalyst particles in situ in the reactor.

2. A process as claimed in claim 1 characterised in that magnetic flocculation of the particles is effected using an electromagnet to apply the magnetic field.

3. A process as claimed in claim 1 or 2 characterised in that the magnetic field applied to the reaction mixture is at least 800 Amperes/metre (10 oersted).

4. A process as claimed in any one of the preceding claims characterised in that the supernatant liquid phase is passed through a "guard" filter upon removal from the process reactor whereby to remove entrained catalyst particles.

**Revendications**

1. Procédé de conduite d'une réaction chimique en phase liquide exécutée dans un réacteur en présence de particules solides d'un catalyseur sur support ou sans support comprenant un métal magnétique ou magnétisable et selon lequel, au terme de la réaction, les particules de catalyseur sont séparées de la phase liquide à l'aide d'un champ magnétique, caractérisé en ce qu'au terme de la réaction:

(1) on fait subir une floculation magnétique aux particules de catalyseur dans le mélange de réaction liquide,
(2) on laisse les particules ainsi floculées se déposer dans le réacteur de manière à produire une phase liquide surnageante, et
(3) on soutire la phase liquide surnageante du réacteur en laissant les particules de catalyseur floculées et déposées in situ dans le réacteur.

2. Procédé suivant la revendication 1, caractérisé en ce qu'on effectue la floculation magnétique des particules à l'aide d'un électro-aimant pour appliquer le champ magnétique.

3. Procédé suivant la revendication 1 ou 2, caractérisé en ce que le champ magnétique appliqué au mélange de réaction est d'au moins 800 ampères/mètre (10 oersted).

4. Procédé suivant l'une quelconque des revendications précédentes, caractérisé en ce qu'on fait passer la phase liquide surnageante à travers un filtre de "garde" lorsqu'elle est évacuée du réacteur de traitement de manière à évacuer des particules de catalyseur entraînées.

**Patentansprüche**

1. Verfahren zur Durchführung einer chemischen Flüssigphasenreaktion, das in einem Reaktionsbehälter in Gegenwart von festen Teilchen eines Katalysators, der ein Trägerkatalysator oder ein trägerfreier Katalysator ist und ein magnetisches oder magnetisierbares Metall enthält, durchgeführt wird und bei dem die Katalysatorteilchen am Ende der Reaktion unter Anwendung eines Magnetfeldes von der Flüssigphase getrennt werden, dadurch gekennzeichnet, daß am Ende der Reaktion

(1) die in der flüssigen Reaktionsmischung enthaltenen Katalysatorteilchen magnetisch ausgeflockt werden,

(2) die auf diese Weise ausgeflockten Teilchen in dem Reaktionsbehälter absitzen gelassen werden, wodurch eine überstehende Flüssigphase erzeugt wird, und

(3) die überstehende Flüssigphase aus dem Reaktionsbehälter abgeleitet wird, wobei ausgeflockte Katalysatorteilchen, die sich abgesetzt haben, in situ in dem Reaktionsbehälter zurückgelassen werden.

2. Verfahren nach Anspruch 1, dadurch gekennzeichnet, daß die magnetische Ausflockung der Teilchen unter Anwendung eines Elektromagneten zum Anlegen des Magnetfeldes durchgeführt wird.

3. Verfahren nach Anspruch 1 oder 2, dadurch gekennzeichnet, daß das an die Reaktionsmischung angelegte Magnetfeld eine Feldstärke von mindestens 800 A/m (10 Oe) hat.

4. Verfahren nach einem der vorhergehenden Ansprüche, dadurch gekennzeichnet, daß die überstehende Flüssigphase bei der Entfernung aus dem bei dem Verfahren angewandten Reaktionsbehälter durch ein "Schutz" filter durchgeführt wird, wobei mitgerissene Katalysatorteilchen entfernt werden.

Fig.1.

Fig.2.

Fig.3.

Fig.4.